# EUROPÄISCHE PATENTSCHRIFT

(11) **EP 1 539 271 B1**
(45) Veröffentlichungstag und Bekanntmachung des Hinweises auf die Patenterteilung: **16.04.2014**
(21) Anmeldenummer: 03807755.8
(22) Anmeldetag: 22.07.2003
(51) Int. Cl.: A61M 1/34, A61M 1/36, A61M 1/16

(54) **BLUTBEHANDLUNGSVORRICHTUNG MIT BLUTRÜCKGABE**
BLOOD TREATMENT DEVICE FOR RETURNING BLOOD
DISPOSITIF DE TRAITEMENT DU SANG PERMETTANT DE RENVOYER DE SANG

(30) Priorität: 11.09.2002 DE 10242008; 30.09.2002 DE 10245619
(43) Veröffentlichungstag der Anmeldung: 15.06.2005
(73) Patentinhaber: Fresenius Medical Care Deutschland GmbH, 61352 Bad Homburg v.d.H. (DE)
(72) Erfinder: BEDEN, Josef, 55252 Mainz-Kastel (DE); MANKE, Joachim, 35792 Löhnberg (DE)
(74) Vertreter: Laufhütte, Dieter
(86) Internationale Anmeldenummer: PCT/EP2003/008000
(87) Internationale Veröffentlichungsnummer: WO 2004/033001

(56) Entgegenhaltungen:
- WO-A-01/51106
- DE-A- 4 240 681
- DE-C- 10 011 208

## Beschreibung

Die Erfindung betrifft eine Blutbehandlungsvorrichtung, vorzugsweise Dialysiervorrichtung, mit den Merkmalen des Oberbegriffs des Anspruchs 1.

Als Blutbehandlungsvorrichtungen sind beispielsweise Dialysiervorrichtungen unterschiedlichster Bauart für verschiedene Anwendungsfälle bekannt. So wird beispielsweise für eine Hämodiafiltration (HDF) ein extrakorporaler Blutkreislauf mit folgenden Bestandteilen geschaffen: mit einem Dialysator, zumindest zwei Leitungen mit Auslässen (Patientennadeln), einer Blutpumpe, einer Dialysatpumpe, einem ersten in der ersten Leitung angeordneten Ventil, einem zweiten in der zweiten Leitung angeordneten Ventil und einem Predilutionsport bzw. einem Postdilutionsport für die Einspeisung der Substituatflüssigkeit.

Die einzelnen Komponenten können hierbei in Differentialbauweise aufgebaut sein. Besonders vorteilhaft sind diese entsprechenden Komponenten aber Bestandteil einer integrierten Kassette, wie sie beispielsweise in der DE 102 24 750.1 vom 04. Juni 2002 beschrieben wurde.

Am Ende einer Dialysesitzung ergibt sich das Problem, dass das im extrakorporalen Kreislauf vorhandene Blut möglichst vollständig an den Patienten zurückgegeben wird. Hierzu sind für die verschiedenen Blutbehandlungsvorrichtungen bereits entsprechende Verfahren bekannt. Ein Verfahren zur Blutrückgabe aus einer Blutbehandlungsvorrichtung ist beispielsweise unter Zitierung von weiteren alternativen Verfahren in der EP 0 578 175 B1 beschrieben.

Die WO 01/51106 A1 beschreibt ein Verfahren und eine Vorrichtung zur Blutrückgabe aus einem extrakorporalen Blutkreislauf. Die Rückgabe des Blutes an den Patienten erfolgt durch Abpumpen mittels der Blutpumpe. Danach wird der Blutkreislauf vom Patienten abgekoppelt, kurzgeschlossen und gespült. Nach der Spülung wird ferner die Spüllösung über den Dialysatkreislauf abgepumpt.

Aus der DE 100 11 208 C1 ist dabei ferner ein Verfahren zum Befüllen und/oder Spülen eines extrakorporalen Kreislaufs eines Hämodialyse- und/oder Hämofiltrationsgerätes sowie ein entsprechendes Blutschlauchset bekannt. Dabei wird vorgeschlagen, nach der Hämodialysebehandlung das distale Ende des Blutzuführleitungsabschnittes von der zwischenzeitlich konnektierten Kanüle dekonnektiert und mit dem zweiten Auslass eines Reinfusionsbeutels zu verbinden. Mit Hilfe der Blutpumpe wird sodann das restliche im extrakorporalen Kreislauf verbliebene Blut zur Rückgabe an den Patienten gefördert, wobei gleichzeitig der Reinfusionsbeutel entleert wird.

Ausgehend von den bekannten Verfahren zur Blutrückgabe aus einer Blutbehandlungsvorrichtung soll eine möglichst noch weiter vereinfachte und zweckmäßiger ausgestaltete Blutbehandlungsvorrichtug für eine möglichst quantitative Blutrückgabe entwickelt werden.

Es ist daher die Aufgabe der vorliegenden Erfindung, eine Blutbehandlungsvorrichtung der eingangs genannten Art in vorteilhafter Art und Weise weiterzubilden.

Diese Aufgabe wird durch eine Blutbehandlungsvorrichtung mit den Merkmalen des Anspruchs 1 gelöst. Vorteilhafte Ausgestaltungen sind Gegenstand der Ansprüche 2 bis 6.

Die Blutrückgabe kann dergestalt sein, dass in einer Blutbehandlungsvorrichtung mit einem Blutbehandlungselement, zwei Leitungen mit Auslässen, einer Blutpumpe, einem ersten in der ersten Leitung angeordneten Ventil, einem zweiten in der zweiten Leitung angeordneten Ventil und einem Predilutionsport für die Einspeisung der Substituatflüssigkeit, mit einer an den Predilutionsport angeschlossenen Substituatversorgungsleitung, in die eine Substituatpumpe geschaltet ist, folgende Schritten durchgeführt werden:
- das erste Ventil in der ersten Leitung wird geöffnet und das zweite in der zweiten Leitung angeordnete Ventil wird geschlossen,
- die Blutpumpe wird auf Durchlaß gestellt oder sie läuft mit, während die Substituatpumpe mittels geförderter Substituatflüssigkeit das Blut verdrängt,
- das Blut wird volumenkontrolliert weiter verdrängt, bis es den Leitungsauslaß der ersten Leitung erreicht hat,
- die Blutpumpe wird geschlossen bzw. gestoppt, das erste Ventil wird geschlossen und das zweite Ventil wird geöffnet,
- die Substituatpumpe verdrängt über geförderte Substituatflüssigkeit Blut durch die freigegebene zweite Leitung sowie das Blutbehandlungselement,
- das Blut wird volumenkontrolliert weiter verdrängt, bis es den Leitungsauslaß der zweiten Leitung erreicht hat.

Die Blutrückgabe kann ferner dergestalt sein, dass in einer Blutbehandlungsvorrichtung mit einem Blutbehandlungselement, zwei Leitungen mit Auslässen, einer Blutpumpe, einem ersten in der ersten Leitung angeordneten Ventil, einem zweiten in der zweiten Leitung angeordneten Ventil und einem Postdilutionsport für die Einspeisung der Substituatflüssigkeit, mit einer an den Postdilutionsport angeschlossenen Substituatversorgungsleitung, in die eine Substituatpumpe geschaltet ist, mit folgenden Schritten durchgeführt wird:
- das erste Ventil in der ersten Leitung wird geöffnet und das zweite in der zweiten Leitung angeordnete Ventil wird geschlossen,
- die Blutpumpe wird auf Durchlaß gestellt oder sie läuft mit, während die Substituatpumpe mittels geförderter Substituatflüssigkeit das Blut durch das Blutbehandlungselement und die erste Leitung verdrängt,
- das Blut wird volumenkontrolliert weiter verdrängt, bis es den Leitungsauslaß der ersten Leitung erreicht hat,
- die Blutpumpe wird geschlossen bzw. gestoppt, das erste Ventil wird geschlossen und das zweite Ventil wird geöffnet,
- die Substituatpumpe verdrängt über geförderte Substituatflüssigkeit Blut durch die freigegebene zweite Leitung,
- das Blut wird volumenkontrolliert weiter verdrängt, bis es den Leitungsauslaß der zweiten Leitung erreicht hat.

Diese Lösungen ermöglichen eine gleichsam arterielle und venöse Blutrückgabe. Im Gegensatz zum Standardverfahren mit arterieller und anschließender venöser Diskonnektion ist die Diskonnektion des Patienten erst ganz am Ende der Rückgabeprozedur notwendig. Dort wird zunächst der arterielle Zugang diskonnektiert und an einen Spüllösungsbeutel, der physiologische Kochsalzlösung enthält, angeschlossen. Mit Hilfe der Blutpumpe wird dann das Blut im Schlauchsystem über den venösen Anschluß an den Patienten zurückgegeben. Die Rückgabe wird dabei manuell überwacht.

Eine Umänderung der Patientenanschlüsse zum Starten der Rückgabe ist beispielsweise nicht notwendig.

Besonders vorteühaft wird die Substituatpumpe zur Flüssigkeitszufuhr verwendet, wodurch eine gute Trenngrenze zwischen Zugabelösung und Blut resultiert.

Gemäß einer bevorzugten Ausgestaltung wird eine hochgenau dosierende Membranpumpe als Substituatpumpe verwendet. Diese ermöglicht in Zusammenwirken mit den beiden optischen Sensoren eine sehr effektive und punktgenaue Blutrückgabe, da das Restvolumen in den Leitungen von den Sensoren bis zum Leitungsauslass bekannt ist und durch entsprechende Ansteuerung der hochgenau dosierenden Membranpumpe dieses bekannte Volumen punktgenau aus der Leitung verdrängt werden kann.

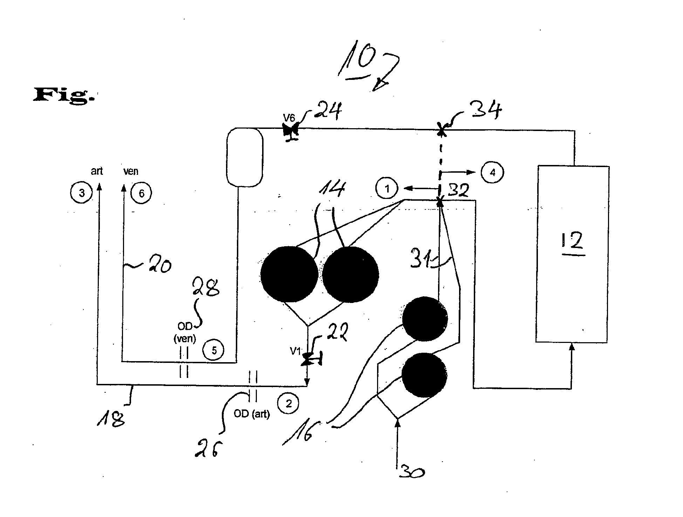

Weitere Einzelheiten, Merkmale und Vorteile der Erfindung werden anhand eines in der Zeichnung dargestellten Ausführungsbeispiels erläutert. Die einzige Figur zeigt ein prinzipielles Schaltbild einer Online Hämodiafiltrationsvorrichtung, mit der das erfindungsgemäße Verfahren durchgeführt werden kann.

Die in der Figur dargestellte Dialysiervorrichtung 10 enthält einen Dialysator 12, eine Blutpumpe 14, die als Membranpumpe ausgebildet ist, eine Substituatpumpe 16, die ebenfalls als Membranpumpe ausgebildet ist, eine erste Leitung 18 als arterielle Blutleitung und eine zweite Leitung 20 als venöse Blutleitung. In der Förderrichtung während einer Blutbehandlung ist in der ersten Leitung 18 stromaufwärts der Blutpumpe 14 ein erstes Ventil 22 angeordnet. Sowohl die Blutpumpe 14 wie die Substituatpumpe 16 sind dabei als parallel geschaltete Doppelpumpe ausgeführt, was eine nahezu gleichmäßige Förderung erlaubt. In der zweiten Leitung 20 ist ein zweites Ventil 24 angeordnet. In der ersten Leitung ist ein erster optischer Detektor 26 und in der zweiten Leitung ist ein zweiter optischer Detektor 28 angeordnet.

Die Substituatpumpe 16 fördert Substituatlösung von einer Substituatquelle 30 über eine Substituatversorgungsleitung 31 entweder zu einem Predilutionsport 32 oder zu einem Postdilutionsport 34. Die Substituatquelle 30 kann ein Beutel mit geeigneter Flüssigkeit oder eine Zubereitungseinheit innerhalb der Dialysiervorrichtung 10 sein, die geeignete Flüssigkeit vor Ort online zubereitet.

Für den Fall, dass die Substituatpumpe 16 das Substituat zu einem Predilutionsport 32 fördert, wird nach Beendigung der Dialyse zur Blutrückgabe aus der Dialysiervorrichtung 10 zum Patienten (hier nicht näher dargestellt) das erste Ventil 22 in der ersten Leitung 18 geöffnet und das zweite Ventil 24 in der zweiten Leitung 20 geschlossen. Das Blut wird weitestgehend aus der Blutpumpe 14 herausgepresst. Die Blutpumpe 14 wird auf Durchlass gestellt oder sie läuft vorzugsweise druckgesteuert mit, während die Substituatpumpe 16 mittels geförderter Substituatflüssigkeit Blut in der Leitung 18 entgegengesetzt zur normalen Strömungsrichtung verdrängt, bis im ersten optischen Detektor 26 festgestellt wird, dass statt Blut Substituatflüssigkeit nachströmt: Dies wird vom optischen Detektor dadurch erkannt, dass die Substituatflüssigkeit entgegen dem Blut wesentlich heller ist.

Das Blut wird von diesem Zeitpunkt an volumenkontrolliert weiter verdrängt, bis es den Leitungsauslass der ersten Leitung erreicht hat. Das Vorlumen in der Restleitung hinter dem ersten Detektor 26 bis zum Leitungsauslass ist genau bekannt und mittels der hochgenau dosierenden Substituatpumpe 16 lässt sich das gewünschte Volumen genau verdrängen. Nach Verdrängen des Volumenanteils an Blut ist das arterielle Blut weitestgehend quantitativ an den Patienten zurückgeführt.

Zum Zurückführen des venösen Bluts wird nun die Blutpumpe geschlossen bzw. gestoppt und das erste Ventil 22 wird geschlossen, während das zweite Ventil 24 geöffnet wird. Die Substituatpumpe verdrängt nun über die geförderte Substituatflüssigkeit Blut durch die freigegebene zweite Leitung 20 sowie den Dialysator 12 bis im zweiten Detektor 28 festgestellt wird, dass statt Blut Substituatflüssigkeit nachströmt. Nun wird das Blut wieder volumenkontrolliert weiter verdrängt, bis es den Leitungsauslass der zweiten Leitung erreicht hat, so dass nun auch das venöse Blut an den Patienten zurückgegeben wurde.

Wird nun die Substituatflüssigkeit nicht durch den Predilutionsport 32, sondern durch den Postdilutionsport 34 zugegeben, so modifiziert sich das vorausgehend beschriebene Verfahren dadurch, dass schon bei der Verdrängung des arteriellen Bluts die Substituatlösung durch den Dialysator 12 läuft und das entsprechende Blut sowie das Blut in der danach folgenden Leitung 18 in der voran beschriebenen Art verdrängt.

Die zuvor dargestellte Reihenfolge des Entleerens der ersten Leitung 18 und anschließend des Entleerens der zweiten Leitung 20 kann im Rahmen der Erfindung natürlich auch vertauscht werden.

## Patentansprüche

1. Blutbehandlungsvorrichtung mit einem Blutbehandlungselement (12), einer Blutpumpe (14), einer Substituatpumpe (16), die über eine Substituatversorgungsleitung (31) Substituatlösung von einer Substituatquelle (30) zu einem Predilutionsport (32) oder Postdilutionsport (34) fördert, einer ersten Leitung (18) als arterieller Blutleitung, einer zweite Leitung (20) als venöser Blutleitung, Ventilen (22, 24) und einer Steuervorrichtung,
**dadurch gekennzeichnet,**
**dass** ein erstes Ventil (22) in der ersten als arteriellen Blutleitung ausgebildeten Leitung (18) stromaufwärts der Blutpumpe (14) und ein zweites Ventil (24) in der zweiten als venöse Blutleitung ausgebildeten Leitung (20) angeordnet sind, wobei nach Beendigung der Dialyse die erste Leitung (18) durch Öffnen des ersten Ventils (22) und Schließen des zweiten Ventils (24) freigebbar ist und wobei die zweite Leitung (20) durch Schließen des ersten Ventils (22) und durch Öffnen des zweiten Ventils (24) freigebbar ist, so dass die Substituatpumpe (16) mittels geförderter Substituatflüssigkeit Blut nach entsprechender Freigabe der jeweiligen Leitung (18 bzw. 20) verdrängen kann.

2. Blutbehandlungsvorrichtung (10) nach Anspruch 1, **dadurch gekennzeichnet, dass** Detektoren (26, 28) in den Leitungen (18, 20) angeordnet sind.

3. Blutbehandlungsvorrichtung (10) nach Anspruch 2, **dadurch gekennzeichnet, dass** die Detektoren (26, 28) optische Detektoren sind.

4. Blutbehandlungsvorrichtung (10) nach einem der vorhergehenden Ansprüche, **dadurch gekennzeichnet, dass** die Pumpen (14, 16) als parallel geschaltete Doppelpumpe ausgeführt sind.

5. Blutbehandlungsvorrichtung (10) nach einem der vorhergehenden Ansprüche, **dadurch gekennzeichnet, dass** die Substituatquelle (30) ein Beutel oder eine Zubereitungseinheit innerhalb der Blutbehandlungsvorrichtung (10) ist.

6. Blutbehandlungsvorrichtung (10) nach einem der vorhergehenden Ansprüche, **dadurch gekennzeichnet, dass** die Blutbehandlungsvorrichtung (10) eine Dialysiervorrichtung (10) ist.

## Claims

1. A blood treatment apparatus comprising a blood treatment element (12), a blood pump (14), a substituate pump (16) which transports substituate solution from a substituate source (30) via a substituate supply line (31) to a predilution port (32) or to a postdilution port (34), a first line (18) as an arterial blood line, a second line (20) as a venous blood line, valves (22, 24) and a control unit,
**characterized in that**
a first valve (22) is arranged upstream of the blood pump (14) in the first line (18) designed as an arterial blood line and a second valve (24) is arranged in the second line (20) designed as a venous blood line, with the first line (18) being able to be released by opening the first valve (22) and closing the second valve (24) after completion of dialysis and with the second line (20) being able to be released by closing the first valve (22) and opening the second valve (24) so that the substituate pump (16) can displace blood by means of the conveyed substituate fluid after a corresponding release of the respective line (18 or 20).

2. A blood treatment apparatus (10) in accordance with claim 1, wherein detectors (26, 28) are arranged in the lines (18, 20).

3. A blood treatment apparatus (10) in accordance with claim 2, wherein the detectors (26, 28) are optical detectors.

4. A blood treatment apparatus (10) in accordance with any one of the preceding claims, wherein the pumps (14, 16) are made as double pumps connected in parallel.

5. A blood treatment apparatus (10) in accordance with any one of the preceding claims, wherein the substituate source (30) is a bag or a preparation unit within the blood treatment apparatus (10).

6. A blood treatment apparatus (10) in accordance with any one of the preceding claims, wherein the blood treatment apparatus (10) is a dialysis apparatus (10).

## Revendications

1. Dispositif de traitement du sang avec un élément de traitement du sang (12), une pompe à sang (14), une pompe à substituat (16), qui, via un conduit d'alimentation de substituat (31) alimente une solution de substituat à partir d'une source de substituat (30) vers un port de prédilution (32) ou un port de postdilution (34), un premier conduit (18) en tant que conduit de sang artériel, un second conduit (20) en tant que conduit de sang vénal, des vannes (22, 24) et un dispositif de commande,
**caractérisé en ce**
**qu'**une première vanne (22) est disposée dans le premier conduit (18) agencé comme conduit de sang artériel en amont de la pompe à sang (14) et une seconde vanne (24) dans le second conduit (20) agencé comme conduit de sang vénal, le premier conduit (18) étant libérable après la fin de la dialyse par l'ouverture de la première vanne (22) et la fermeture de la seconde vanne (24), et le second conduit (20) étant libérable par la fermeture de la première vanne (22) et par l'ouverture de la seconde vanne (24), de sorte que la pompe à substituat (16) puisse refouler du sang à l'aide du liquide de substituat transporté après la libération correspondante du conduit respectif (18 et/ou 20).

2. Dispositif de traitement du sang (10) selon la revendication 1, **caractérisé en ce que** des détecteurs (26, 28) sont disposés dans les conduits (18, 20).

3. Dispositif de traitement du sang (10) selon la revendication 2, **caractérisé en ce que** les détecteurs (26, 28) sont des détecteurs optiques.

4. Dispositif de traitement du sang (10) selon une quelconque des revendications précédentes, **caractérisé en ce que** les pompes (14, 16) sont agencées comme pompe double raccordée en parallèle.

5. Dispositif de traitement du sang (10) selon une quelconque des revendications précédentes, **caractérisé en ce que** la source de substituat (30) est un sachet ou une unité de préparation au sein du dispositif de traitement du sang (10).

6. Dispositif de traitement du sang (10) selon une quelconque des revendications précédentes, **caractérisé en ce que** le dispositif de traitement du sang (10) est un dispositif de dialyse (10).
